# EUROPEAN PATENT APPLICATION

(11) **EP 3 919 005 A1**
(43) Date of publication of application: **08.12.2021**
(21) Application number: 20305600.7
(22) Date of filing: 05.06.2020
(51) Int. Cl.: A61B 17/11, A61B 17/00

(54) **DEVICE FOR NERVE REPAIR**

(71) Applicant: Université de Strasbourg, 67000 Strasbourg (FR); Centre National de la Recherche Scientifique, 75016 Paris (FR); Hôpitaux Universitaires de Strasbourg (HUS), 67000 Strasbourg (FR)
(72) Inventor: SALAZAR BOTERO, Santiago, 660003 PEREIRA (CO); BAHLOULI, Nadia, 67170 WINGERSHEIM (FR); LIVERNEAUX, Philippe, 67000 STRASBOURG (FR); FACCA, Sybille, 67000 STRASBOURG (FR)
(74) Representative: Lavoix

(57) **Abstract**

The present invention relates to concerns a new type of device for nerve repair and a method for repairing severed nerves, and in particular relates to a device for nerve surgery of a nerve comprising a proximal stump and a distal stump, said device comprising a tube, called bridge tube, comprising a proximal opening and a distal opening, wherein the proximal stump could be inserted in said proximal end, wherein the distal stump could be inserted in said distal end, and wherein said bridge tube comprises an opening, called manipulation opening, suitable for inserting a tool in the manipulation opening for manipulating the proximal stump and the distal stump and thereby inserting said nerve stumps into said bridge tube, wherein said device comprises a cover for closing said manipulation opening.

## Description

The present invention concerns a new type of device for nerve repair and a method for repairing severed nerves.

### State of the art

When a nerve axon is severed, the part still attached to the cell body. This part of the nerve is called the proximal stump and its extremity is called the proximal end, while the other nerve part is called the distal stump and its extremity is called the distal end.

To regenerate the severed nerve, the surgeon needs to put the nerve extremities in correct place, i.e. in front of each other, to correctly suture the nerve extremities and provide a good regeneration of the nerve.

Of course, this surgical act needs a very high degree of competences and training. The success of this surgical act highly depends on the nerve manipulation. Surgeons need devices for an easier nerve manipulation to improve the surgery and the regeneration of the severed nerve.

Nerve repair can be performed in two ways, either by direct suturing, by adding autograft, allograft or by interposing a nerve regeneration guide. Regardless of the method chosen, the nerve must be sutured between itself or with the allograft, autograft or nerve regeneration guide, this suture requires specialized instruments, optical magnification and microsurgical training. In case of nerve regeneration guide, the surgeon put the extremity of both nerves ends in a single tube to suture the nerve extremities. Typically, the both nerves stumps (proximal and distal stumps) are introduced in a single tube so that each extremity of both nerve stumps is maintained in front of each other while enabling suturing and nerve regeneration to be again functional. The space between the nerve stumps covered by the nerve guide is called the regeneration chamber. This site is where axons grow from the proximal stump to the distal stump and where neurotrophic substances could be introduced to enhance nerve regeneration.

The manipulation, despite all careful manipulation by the surgeon, could be more or less damaged in addition to damages caused by suturing nerve extremities. Nerve suture is a complicated technique of surgery (microsurgery).

One problem of nerve guides is that the surgeon still needs to perform a suture of nerve ends.

Nerve suture occupies space inside the regenerating nerve and induces inflammation around it. This solution could prevent the nerve regeneration. It is also widespread demonstrated that sutures cannot withstand physiological forces on nerves which limits movement during rehabilitation of the limb.

US 2004/0186488 A1 relates to a method of peripheral nerve or blood vessel reconstruction requiring the use of a unique connector. The method employs negative gauge pressure, applied through a port on the connector, to draw the ends of the disrupted nerve or vessel into the connector. Next, a biocompatible adhesive is used to cement near the ends of the nerve or vessel circumferentially to the inside of the connector wall, leaving the cut ends touching each other but free of the bio-adhesive. Suction is applied to the umbilical port as the cut ends of the microstructure are introduced at both ends of the main conduit. In such a device, no manipulation tools (typically ancillary tools) can be introduced in the umbilical port because of the design of the umbilical port.

### Goals of the invention

The invention aims to solve one or more, preferably all, of the above-recited technical problems. The present invention focuses on nerve regeneration guide.

In particular, the invention aims to solve the technical problem of providing a device for limiting negative impact of nerves manipulation on nerves in need of surgery, and in particular limiting damages to nerves in need of surgery, in particular of severed nerves.

In particular, the invention aims to solve the technical problem of providing a device for limiting the time for a surgeon to put extremity of severed nerves in correct place for regeneration of the nerve.

In particular, the invention aims to solve the technical problem of providing a device for regenerating a severed nerve with better quality.

In addition, the invention aims to solve the technical problem of providing a device allowing the use of ancillary tools for nerve surgery.

In addition, the invention aims to solve the technical problem of providing a device with less inconvenient for the patient once the operation is completed.

In particular, the invention aims to provide a device, which can be manufactured at an industrial scale.

### Description of the invention

The present invention relates to a device for nerve surgery of a nerve comprising a proximal stump and a distal stump, said device comprising a tube, called bridge tube, comprising a proximal opening and a distal opening, wherein the proximal stump could be inserted in said proximal end, wherein the distal stump could be inserted in said distal end, and wherein said bridge tube comprises an opening, called manipulation opening, suitable for inserting a tool in the manipulation opening for manipulating the proximal stump and the distal stump and thereby inserting said nerve stumps into said bridge tube, wherein said device comprises a cover for closing said manipulation opening.

The device for nerve surgery according to the present invention is a new nerve repair system representing a technological breakthrough with respect to current clinical practice.

The present invention has the great advantage of avoiding the epiperineural suture.

Typically, said bridge tube comprises a regeneration chamber, wherein the proximal and distal stumps regenerate in said regeneration chamber.

In one embodiment, said bridge tube comprises a regeneration chamber in front of the manipulation opening.

Advantageously, neurotrophic and angiogenic growth factors embedded in microsphere or hydrogel are injected through the window of regeneration chamber to the regeneration chamber This involves a huge advance in the way of nerve repair.

Advantageously, said cover is a cover tube having an inner diameter suitable for inserting said bridge tube into said cover tube.

In one embodiment, said cover is a patch having dimensions covering at least said manipulation opening.

In one embodiment, said cover is tape having dimensions covering at least said manipulation opening.

In one embodiment, said bridge tube has an inner diameter for receiving a nerve, typically a peripheral nerve, in particular a nerve selected from the group consisting of a digital nerve stump, including a nerve of thumb, index, middle, right finger, left finger, or little finger, brachial plexus nerve, sciatic nerve, median ulnar nerve.

Typically, the external diameter of said bridge tube is smaller than the inner diameter of said cover tube and has dimensions allowing the bridge tube to be introduced into said cover tube and keeping the bridge tube in place in the cover tube.

In one embodiment, said bridge tube has a length of 20 to 80 mm.

In one embodiment, said bridge tube has an inner diameter of 1.1 to 10.6 mm.

In one embodiment, said manipulation opening has a length of less than the bridge tube length and preferably less than 3.5 mm if the bridge tube is longer than 3.5 mm.

In one embodiment, said manipulation opening has a length between 0,25-0,5 of the length of the bridge tube and a diameter between 0,3-0,8 of the diameter of the bridge tube.

In one embodiment, said cover tube has a length less than the length of the bridge tube, typically, the length of said cover tube is of 15 to 55 mm. Typically, the length of the cover tube is equal or less than 80% of the length of the bridge tube.

In one embodiment, said cover tube has an inner diameter longer than the outer diameter of the bridge tube. Typically, the inner diameter of said co.r tube is of 105% to 120% of the outer diameter of the bridge tube.

In one embodiment, the inner diameter of said cover tube is of 2.2. to 11.7 mm.

In one embodiment, said patch has dimensions equal to or of +20% more than the dimensions of said manipulation opening. Typically, the surface in contact between the patch and the external wall of the bridge tube is sufficient for gluing the patch to the bridge tube.

In one embodiment, said tape has a width of +20% more than the width of said manipulation opening.

In one embodiment, said tape has a length of +11% to +50% more than the perimeter of said bridge tube.

In one embodiment, said bridge tube and/or said cover are biodegradable.

Said bridge tube is preferably made of a composite material, all of which are absorbable, biocompatible materials.

Preferably, the tubes are bioresorbable within less than 8 months, typically from 3 to 6 months. Bioresorbable means that the tube is fully degraded in vivo.

Advantageously, said bridge tube and/or cover comprises or consists of polycaprolactone, polyhydroxyalkanoate (PHA), polylactic acid (PLA), poly(lactic-coglycolic) acid (PLGA), polyglycolic acid (PGA), polycaprolactone (PCL), adipic acid, aminocaproic acid, poly (butylene succinate), chitosan, collagen or a derivative or a combination thereof.

In one embodiment, said bridge tube comprises or consists of microfibers.

In one embodiment, the bridge tube comprises or consists of epsilon polycaprolactone (example: monocryl®).

In one embodiment, the bridge tube comprises fibers embedded in a polymer matrix forming the wall of the bridge tube. In one embodiment, said fibers are aligned longitudinally in the bridge tube. Advantageously, this allows a better resistance to nerve movements.

Advantageously, the fibers provide strength comparable to that of healthy nerves.

In one embodiment, a polymer suitable for bridge and cover tubes of the invention is sprayable or electrospinnable or another method known from the skilled person.

Typically, the tube has an extruded, woven, non-woven, braided, knitted, printed, in particular 3D printed, or another structure.

The polymer to be sprayed is preferably in a liquid form, preferably in solution. In one embodiment, the polymer is dissolved in a solvent before spraying. For example, a solvent is chloroform.

In one embodiment, a polymer suitable for a bridge or cover tube of the invention is electrospinable (can be electrospinated).

In one embodiment, the bridge tube and/or the cover tube are sterile.

In one embodiment, the tube present pores. For example, the pore size is such that the material is impermeable to fibroblasts and such that the material is permeable to ion exchange to retain nerve hydration. Typically, the pore size is 10 µm to 300 µm.

Advantageously, the tube, in particular the bridge tube, is sufficiently flexible to facilitate the aspiration of the nerve.

Advantageously, the tube, in particular the bridge tube, is rigid enough not to collapse.

In one embodiment, said bridge tube and/or said cover are flexible to follow the movement of the nerve in the body of a patient.

In one embodiment, the aspiration instrument set comprises or consists of one non-beveled needles. Preferably, the needles are non-beveled.

Typically, a catheter or a needle has a size from 24G to 14G depending on the size of the nerve involved.

Typically, a needle or catheter is coupled with a manual or automated aspiration system to control when the nerve must be sucked in.

Typically, to suck the nerve a pump, such as for example a syringe, is used.

In one embodiment, said device comprises glue to glue a part of said nerve stumps thereby solidarizing the nerve proximal stump and the nerve distal stump to the wall of said distal bridge tube. Any type of glue suitable for nerve repair may be used. The skilled person knows such the glues, in particular surgical glues.

In one embodiment, the glue is a cyanoacrylate. For example, the glue is a Octyl-2-cyanoacrylate (Dermabond; Ethicon, Inc, Somerville, NJ) or Butyl cyanoacrylate Histoacryl; B. Braun, Melsungen, Germany; or IFABOND® de Peters Surgical.

In one embodiment, the glue is a Polyethylene Glycol Hydrogel. In one embodiment, the glue is a polyethylene glycol (Duraseal®, Integra).

In one embodiment, the glue is a fibrin glue (for example: a combination of a human Fibrinogen (as coagulable protein) and human thrombin such as Tisseel® or Tissucol®).

Advantageously, the present invention allows repairing a nerve without using technics of suture of microsurgery, without the need for microsurgical training, without the need for optical magnification and with a resistance better than a sutured nerve or equivalent to the nerve guides.

Advantageously, the device of the invention allows improving the surface of contact solidarizing a nerve stump to the bridge tube wall.

The present invention also relates to a device according to the invention, or a composition for manufacturing said device, wherein said device is for use in a method for nerve repair, said method comprises introducing the proximal stump in said proximal end of the bridge tube by manipulating tools introduced in the manipulation opening, introducing the distal stump in said distal end of the bridge tube by manipulating tools introduced in the manipulation opening, (i) gluing said proximal stump to an inner wall of said bridge tube, and/or (ii) gluing said distal stump to an inner wall of said bridge tube, wherein said method comprises closing the manipulation opening by said cover.

Advantageously said proximal stump and said distal stump are manipulated through said manipulation opening, for example by aspiration of nerve stumps or extremities.

The invention also relates to a method for nerve repair, said method comprises introducing the proximal stump in said proximal end of the bridge tube by manipulating tools introduced in the manipulation opening, introducing the distal stump in said distal end of the bridge tube by manipulating tools introduced in the manipulation opening, (i) gluing said proximal stump to an inner wall of said bridge tube, and/or (ii) gluing said distal stump to an inner wall of said bridge tube, wherein said method comprises closing the manipulation opening by said cover.

In one embodiment, said tool is an ancillary tool, for example comprising an aspiration instrument set for sucking a nerve stump and manipulating the nerve stump through said manipulation opening.

According to a method of the present invention, a surgeon needs only from 1 to 5 minutes, typically not more than 3 minutes to join the nerve proximal stump and the nerve distal stump whereas typically more than 10 minutes are needed if a suture is performed.

Advantageously, no exogenous substance is introduced in the repaired nerve according to the invention, whereas the suture introduces an exogenous body in the nerve according to the prior art.

Advantageously, nerve manipulation is easier according to the present invention whereas it requires strong surgeon training according to the prior art.

In one embodiment, the nerve proximal stump (extremity) is first sucked by an aspiration instrument through the proximal end of the bridge tube,.

In one embodiment, the nerve distal stump (extremity) is first sucked by an aspiration instrument through the distal end of the bridge tube.

In one embodiment, the one nerve stump is placed in the cover tube prior to placing the bridge tube.

In one embodiment, the cover tube is placed in a position fully covering the manipulation opening of said bridge tube, after nerve aspiration. Typically, the cover tube is inserted and slid onto a nerve stump a few cm upstream before placing the bridge tube; both stumps are introduced in the bridge tube by manipulating said manipulation tools inserted into said manipulation opening; both stumps are glued onto the inner wall of the bridge tube, then the cover tube is slid onto the bridge tube and glued to the bridge tube, making sure to obliterate the manipulation opening.

In one embodiment, both stumps are first introduced in the bridge tube by manipulating said manipulation tools inserted into said manipulation opening; both stumps are glued onto the inner wall of the bridge tube; and then the patch is applied to cover the manipulation opening, which is glued all around.

In one embodiment, the tape is first positioned on the bridge tube; both stumps are then introduced in the bridge tube by manipulating said manipulation tools inserted into said manipulation opening; both stumps are glued onto the inner wall of the bridge tube; and finally, the tape is rolled up around the bridge tube to cover the manipulation opening and glued on itself.

In one embodiment, the proximal stump is glued on the inner wall of said bridge tube. In another embodiment, the distal stump is glued on the inner wall of said bridge tube. In another embodiment, glue is placed on the aspiration instrument extremity in contact with said nerve proximal or distal stump to glue the nerve stumps.

In another embodiment, glue is placed on the inner wall of the bridge tube to solidarize the proximal stump to the bridge tube. In another embodiment, glue is placed at the extremity of the cover tube to solidarize the bridge tube containing the two stumps to the cover tube.

In another embodiment, before gluing the cover tube and the bridge tube together the regeneration chamber of the bridge tube is filled with available stem cells, neurotrophic and angiogenic growth factors or scaffolds (hydrogel, microspheres ...).

Advantageously, the nerve stumps are physically manipulated by ancillary tools through the manipulation opening. Typically, by such manipulation, each stump is put in place in the bridge tube sequentially. The ancillary tool is introduced in the manipulation opening to come out from one end of the bridge tube and be in contact with a nerve stump. Then the nerve stump is introduced by the ancillary tool in the bridge tube. The nerve stump is typically glued to the inner wall of the bridge tube. The ancillary tool can be withdrawn from the manipulation opening after putting in place the nerve stump.

In the description, the fist stump refers either to the proximal stump or the distal stump and the second stump refers to the other respective stump, i.e. the distal stump or the proximal stump, respectively.

In an embodiment, a first nerve stump is drawn by an ancillary tool having an aspiration means and the first nerve stump is glued to the inner wall of the bridge tube. Then, the second nerve stump is then drawn by an ancillary tool having an aspiration means through the end of the bridge tube opposite to the end in which the first nerve stump was introduced.

In one embodiment, the nerve proximal and distal stumps are in contact, typically, if no nerve substance was lost by nerve injury.

In one embodiment, the nerve proximal and distal stumps are separated by a distance inferior to the length of the bridge tube.

In one embodiment, wherein a cover tube is implemented, the present invention device works as a plug and play system, very easy to manipulate, thereby improving the surgical steps for nerve repair.

In one embodiment, the bridge tube and/or the cover tube is coated at least in part on its inner surface or wall by one or more active ingredients.

In one embodiment, such active ingredient improves nerve repair or regeneration.

In one embodiment, such active ingredient improves the solidarizing of the nerve to the tube wall.

In one embodiment, such active ingredient modifies the bio-resorption of the tube.

In one embodiment, such active ingredient improves the biocompatibility of the tube with the nerve or body in which it is introduced, and in particular said active ingredient limits and preferably avoids immune reaction.

In one embodiment, drugs, scaffolds and/or stem cells are introduced in the regeneration chamber.

In one embodiment, said bridge tube and/or cover tube is/are formed by 3D printing.

In one embodiment, said bridge tube and/or cover tube is/are formed by electrospinning or jetspraying.

In one embodiment, said bridge tube and/or cover tube is/are formed by molding.

In one embodiment, the mold is a hollow right circular cylinder having an inner diameter of the larger radius corresponding to the outer diameter of the bridge tube and an outer diameter of the smaller radius corresponding to the inner diameter of the bridge tube.

Typically, the mold for the cover tube is a right circular cylinder having an outer diameter corresponding to the inner diameter of the manufactured tube.

In one embodiment, the mold is a rotating cylinder.

If a cover tube is implemented, in one embodiment the cover tube is fabricated with a slightly bigger diameter. This cover tube will shelter the bridge tube and close the manipulation opening. Preferably, the two tubes fit together and are hold together.

In one embodiment, the tubes are fabricated by a spraying the composition or a part thereof.

In one embodiment, a tube is prepared by spraying a polymer, preferably a biocompatible polymer, on a mold allowing forming said tube.

In one embodiment, preferably for manufacturing the bridge tube, fibers are deposited in the mold randomly with the length parallel to the tube revolution axis direction thereby providing longitudinal fibers. Then, a polymer layer is sprayed on the longitudinal fibers.

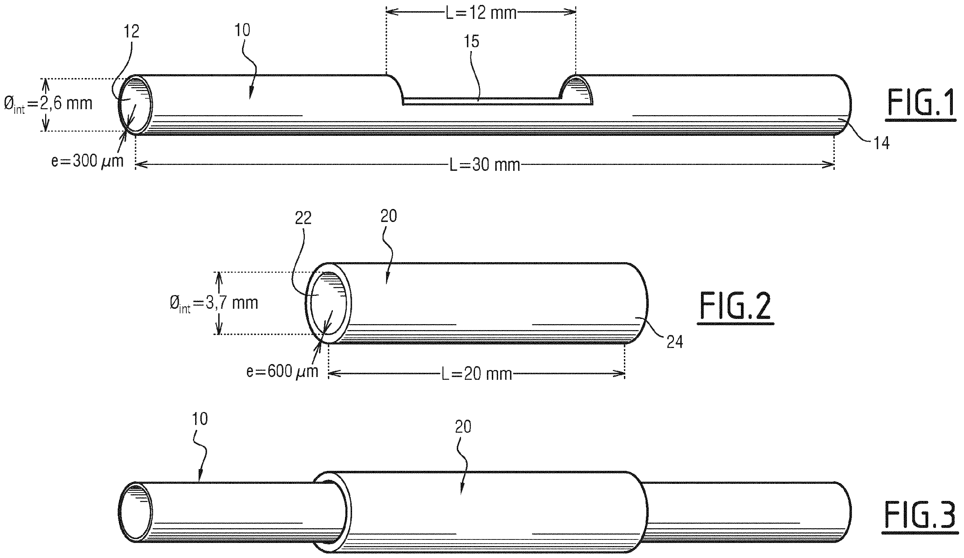

In the figures
Figure 1 represents an embodiment of a bridge tube according to the present invention. Dimensions are given for illustrative purpose.
Figure 2 represents an embodiment of a cover tube according to the present invention. Dimensions are given for illustrative purpose.
Figure 3 represents an embodiment of a bridge tube and a cover tube according to the present invention wherein the cover tube cover the manipulation opening of the bridge tube8
Figure 4 to 17 represents an embodiment with a cover tube of the method of the present invention for inserting nerve stumps into the bridge tube.

### Examples

### Example 1 - Bridge tube with cover tube

The bridge tube and the cover tube are nonwovens made by electrospinning 100% monofilaments ε-polycaprolactone (possibility of chloroform residue, used as a solvent for electrospinning). The bridge tube includes in addition a 5-0 Monocryl monofilament (100µm to 150µm diameter), consisting of poliglecaprone 25, a copolymer of glycoside and ε-polycaprolactone.

The structure has a pore size equivalent from 10 to 300 microns and is slightly translucent when wet.

The tubes are:
- Bioresorbable within 6-12 months (18 months maximum).
- Bioactive
- Biocompatible
- and do not induce inflammatory reactions.

The adhesive used is an octyl-2- cyanoacrylate glue or IFABOND® and is biocompatible.

The bridge tube has a flexibility allowing a good handling during the operation, i.e., t is easy to bend so that the nerve ends can be inserted more easily without losing its cylindrical structure (the walls do not touch).
- The cover tube has a greater rigidity than the bridge tube due to a thickness of the wall more important.

The integral structure (bridge tube + cover tube + glue) allows resisting a tensile force from 7 to 12 N, i.e. a force greater than that of rupture of sutured hand nerve stumps (hand nerve) (F_{rupt nerve hand} = 2.2 N), and even greater than the maximum force measured in the hand on healthy nerves (F_{max appl} = 5.2 N).

Both tubes are sterilized with ethylene oxide.

By reference to the figures:

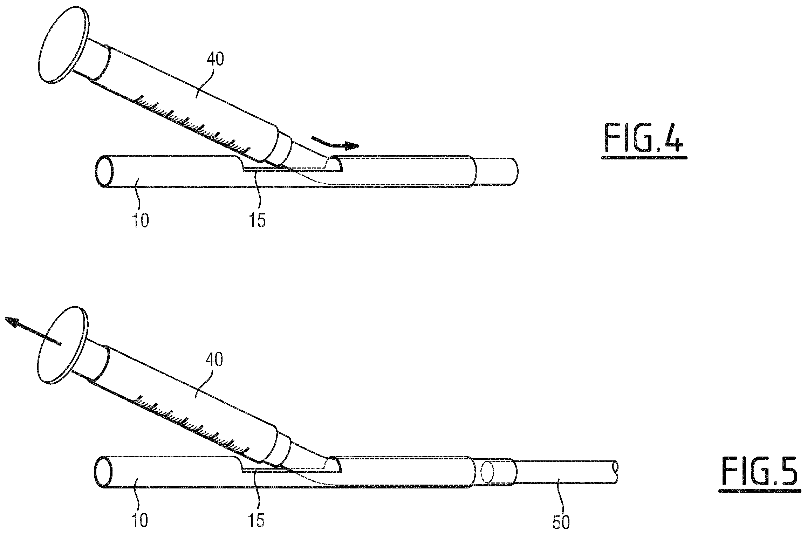

Figure 4: Step 1 - Passing an ancillary tool 40 with an aspiration means (syringe with tip) through the manipulation opening 15 of a bridge tube 10. Said ancillary tool 40 is introduced in the bridge tube 10 up to the end 14 of the bridge tube 10.
Figure 5: Step 2 - Then, the first nerve stump 50 is sucked by the ancillary tool 40.

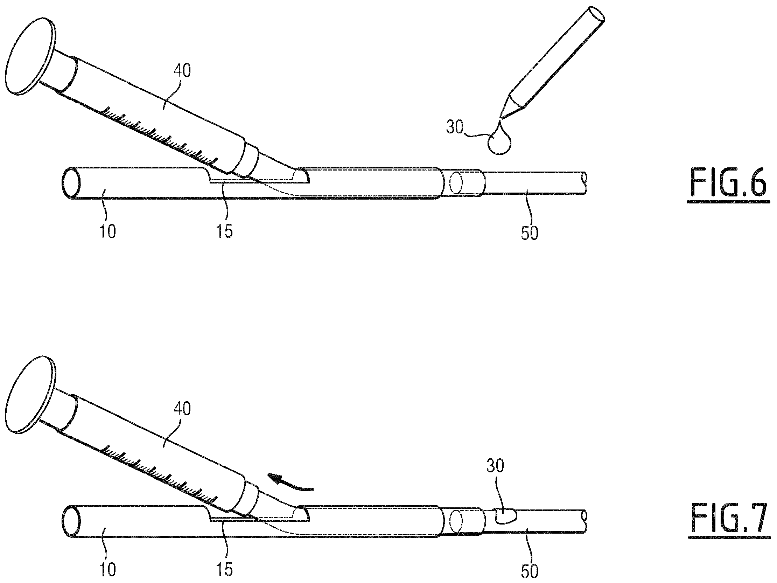

Figures 6: Step 3 - Then a drop of glue 30 is applied to the accessible stump of the nerve.
Figure 7: Step 4 - The ancillary tool 40 and the nerve stump 50 are slid in the bridge tube 10 guide.

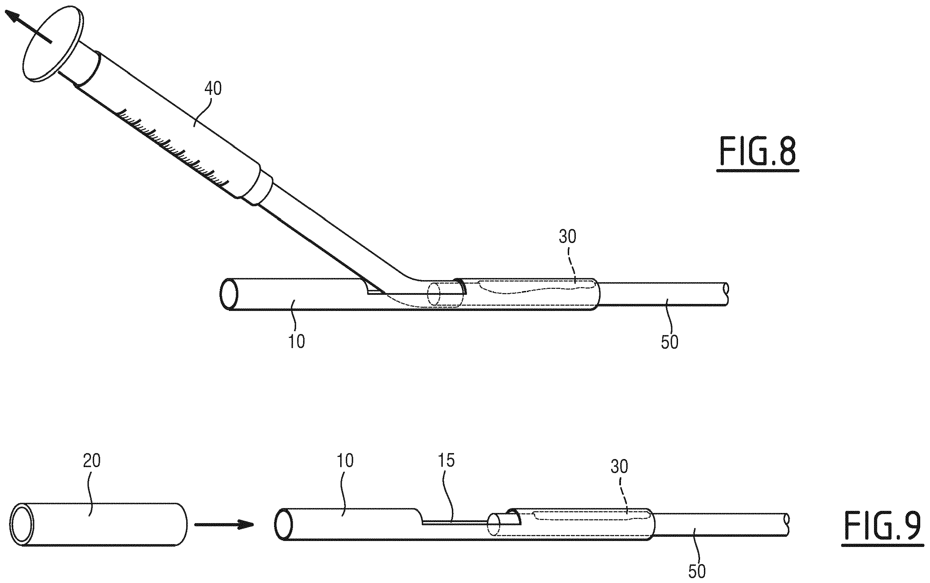

Figure 8: Step 5 - Then the nerve stump 50 is put in place in the bridge tube 10 and the ancillary tool 40 removed from the manipulation opening 15. Release the nerve.
Figure 9: Step 6 - The cover tube 20 is inserted along the bridge tube 10 attached to the nerve stump 50.

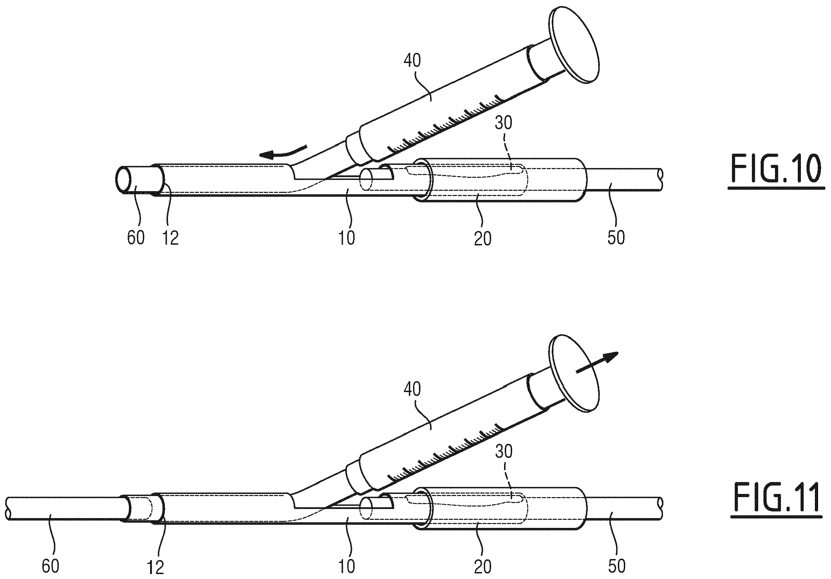

Figure 10: Step 7 - Step 1 according to figure 4 is repeated with the second stump 60. The ancillary tool 40 is introduced in the bridge tube 10 through the manipulation opening 15 up to the end 12 of the bridge tube 10.
Figure 11: Step 8 - Step 2 according to figure 5 is repeated.

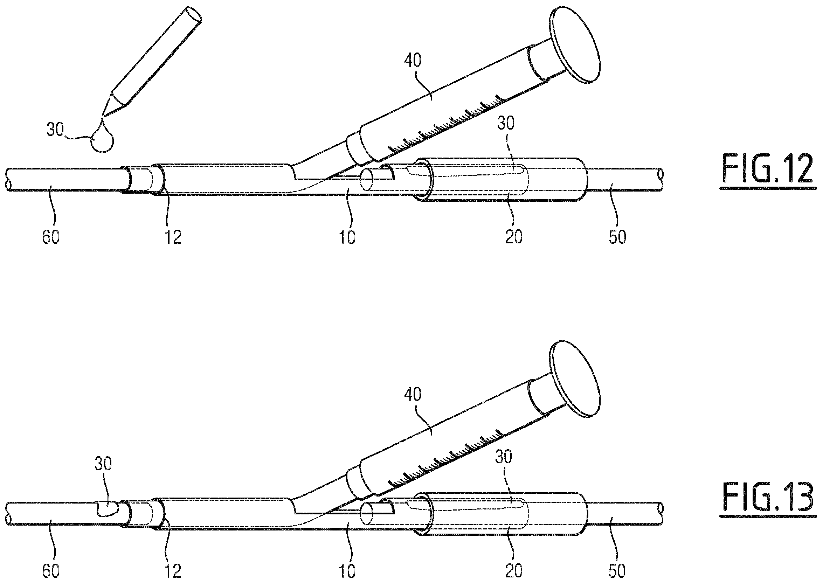

Figures 12 and 13: Step 9 - Then a drop of glue 30 is applied to the accessible stump of the nerve 60.

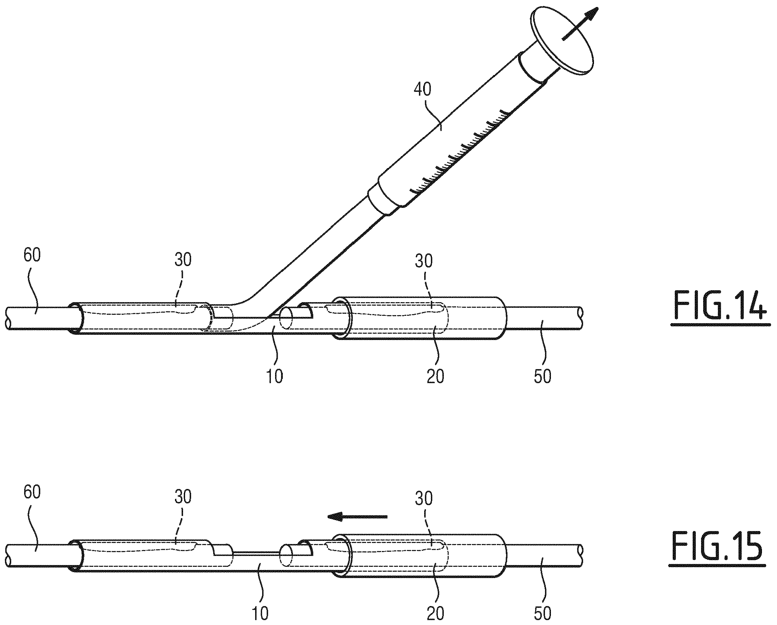

Figure 14: Step 10 - The ancillary tool 40 is withdrawn from the bridge tube 10 through the manipulation opening 15.
Figure 15: Step 11 - Then the cover tube 20 is slide along the bridge tube 10 so that the cover tube 20 completely covers the manipulation opening 15.

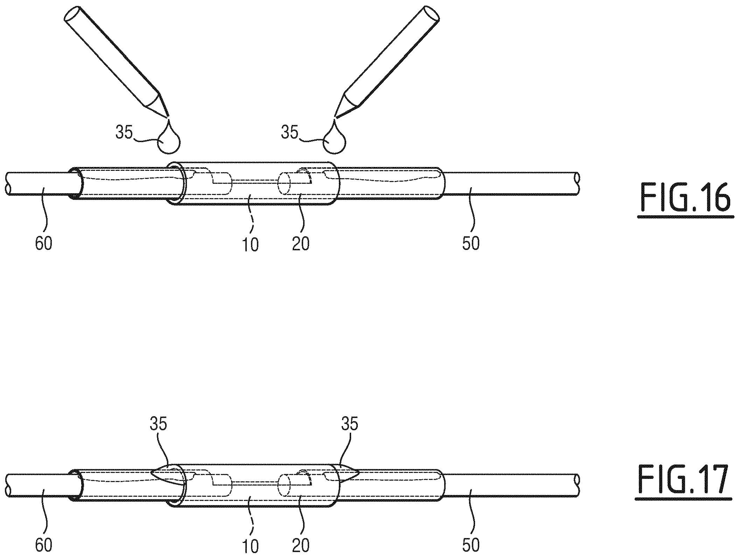

Figure 16: Step 12 - Fibrin is applied as sealant to both ends 22, 24 of the cover tube 20 to seal its interface with the bridge tube 10.
Figure 17: An embodiment of a system 1 is illustrated, said system 1 including a bridge tube 10, a cover tube 20 solidarized by the sealant 35 to the bridge tube 10, said bridge tube 10 including the two nerve stumps 50,60 in place for their regeneration.

## Claims

1. Device for nerve surgery of a nerve comprising a proximal stump and a distal stump, said device comprising a tube, called bridge tube, comprising a proximal opening and a distal opening, wherein the proximal stump could be inserted in said proximal end, wherein the distal stump could be inserted in said distal end, and wherein said bridge tube comprises an opening, called manipulation opening, suitable for inserting a tool in the manipulation opening for manipulating the proximal stump and the distal stump and thereby inserting said nerve stumps into said bridge tube, wherein said device comprises a cover for closing said manipulation opening.

2. Device according to claim 1, wherein said cover is a cover tube having an inner diameter suitable for inserting said bridge tube into said cover tube.

3. Device according to claim 1, wherein said cover is a patch having dimensions covering at least said manipulation opening.

4. Device according to claim 1, wherein said cover is tape having dimensions covering at least said manipulation opening.

5. Device according to any one of claims 1 to 4, wherein said device comprises glue to glue a part of said nerve stumps thereby solidarizing the nerve proximal stump and the nerve distal stump to the wall of said distal bridge tube.

6. Device according to any one of claims 1 to 5, wherein said bridge tube and/or said cover are biodegradable.

7. Device according to any one of claims 1 to 6, wherein said bridge tube and/or said cover are flexible to follow the movement of the nerve in the body of a patient.

8. Device according to any one of claims 1 to 7, wherein said bridge tube and/or said cover comprises or consists of polycaprolactone, polyhydroxyalkanoate (PHA), polylactic acid (PLA), poly(lactic-co-glycolic) acid (PLGA), polyglycolic acid (PGA), polycaprolactone (PCL), adipic acid, aminocaproic acid, poly (butylene succinate), chitosan, collagen or a derivative or a combination thereof.

9. Device according to any one of claims 1 to 8, wherein said tool is an ancillary tool, for example comprising an aspiration instrument set for sucking a nerve stump and manipulating the nerve stump through said manipulation opening.

10. Device according to any one of claims 1 to 9, wherein said bridge tube comprises a regeneration chamber in front of the manipulation opening.

11. Device according to any one of claims 1 to 10, wherein said bridge tube has an inner diameter for receiving a nerve, typically a peripheral nerve, in particular a nerve selected from the group consisting of a digital nerve stump, including a nerve of thumb, index, middle, right finger, left finger, or little finger, brachial plexus nerve, sciatic nerve, median ulnar nerve.

12. A device according to any one of claims 1 to 11, or a composition for manufacturing said device, wherein said device is for use in a method for nerve repair, said method comprises introducing the proximal stump in said proximal end of the bridge tube by manipulating tools introduced in the manipulation opening, introducing the distal stump in said distal end of the bridge tube by manipulating tools introduced in the manipulation opening, (i) gluing said proximal stump to an inner wall of said bridge tube, and/or (ii) gluing said distal stump to an inner wall of said bridge tube, wherein said method comprises closing the manipulation opening by said cover.

13. The device for nerve repair according to claim 12, wherein said proximal stump and said distal stump are manipulated through said manipulation opening, for example by aspiration of nerve stumps or extremities.

14. A method for nerve repair, said method comprises introducing the proximal stump in said proximal end of the bridge tube by manipulating tools introduced in the manipulation opening, introducing the distal stump in said distal end of the bridge tube by manipulating tools introduced in the manipulation opening, (i) gluing said proximal stump to an inner wall of said bridge tube, and/or (ii) gluing said distal stump to an inner wall of said bridge tube, wherein said method comprises closing the manipulation opening by said cover.
